**Europäisches Patentamt**

**European Patent Office**

**Office européen des brevets**

(19)

(11) Veröffentlichungsnummer: **0 022 510**
**A1**

(12) ## EUROPÄISCHE PATENTANMELDUNG

(21) Anmeldenummer: **80103718.5**

(22) Anmeldetag: **01.07.80**

(51) Int. Cl.³: **C 07 C 11/09**, C 07 C 7/148,
C 07 C 1/24, C 07 C 41/06

(30) Priorität: **14.07.79 DE 2928510**

(43) Veröffentlichungstag der Anmeldung: **21.01.81**
**Patentblatt 81/3**

(84) Benannte Vertragsstaaten: **AT BE CH DE FR GB IT LI LU
NL SE**

(71) Anmelder: **BASF Aktiengesellschaft,
Carl-Bosch-Strasse 38, D-6700 Ludwigshafen (DE)**

(72) Erfinder: **Brunner, Erwin, Dr., Neuhausener Weg 1,
D-6700 Ludwigshafen (DE)**
Erfinder: **Schubert, Eckart, Dr., Keplerstrasse 30,
D-6900 Heidelberg (DE)**
Erfinder: **Lindner, Alfred, Dr., Ringstrasse 22,
D-6712 Bobenheim-Roxheim 1 (DE)**
Erfinder: **Merger, Franz, Dr., Max-Slevogt-Strasse 25,
D-6710 Frankenthal (DE)**
Erfinder: **Volkamer, Klaus, Dr., Heidelberger Ring 21,
D-6710 Frankenthal (DE)**
Erfinder: **Sandrock, Gerhard, Dr.,
Albrecht-Duerer-Ring 36C, D-6710 Frankenthal (DE)**
Erfinder: **Strohmeyer, Max, Dr., Woogstrasse 41,
D-6703 Limburgerhof (DE)**

(54) **Verfahren zur Gewinnung von Isobuten aus Isobuten enthaltenden C4-Kohlenwasserstoffgemischen.**

(57) Verfahren zur Gewinnung von Isobuten aus Isobuten enthaltenden $C_4$-Kohlenwasserstoffgemischen durch Umsetzung des Gemisches mit einem primären Alkohol in Gegenwart eines sauren Kondensationsmittels und Zerlegung des gebildeten tertiären Äthers in Gegenwart eines sauren Katalysators bei erhöhten Temperaturen, bei dem man als primären Alkohol einen primären $C_3$- oder $C_4$-Alkohol und als saures Kondensationsmittel für die Ätherbildung Ionenaustauscher in der Wasserstofform verwendet, wobei man den primären $C_3$- oder $C_4$-Alkohol und das $C_4$-Kohlenwasserstoffgemisch, gegebenenfalls nach vorheriger Vermischung, zunächst der Reaktionszone (4) für die Verätherung, in der sich der Ionenaustauscher befindet, zuführt, das aus der Reaktionszone (4) für die Verätherung erhaltene Reaktionsgemisch anschliessend in einer ersten Destillationszone (6) destilliert, als Kopfprodukt ohne Einschaltung einer Wasserwäsche ein die nicht umgesetzten Kohlenwasserstoffe enthaltendes $C_4$-Kohlenwasserstoffgemisch mit einem Gehalt an primären $C_3$- oder $C_4$-Alkohol von höchstens 1000 Gewichts-ppm und als Bodenprodukt den gebildeten, gegebenenfalls noch im Überschuss zugesetzten primären $C_3$- oder $C_4$-Alkohol enthaltenden $C_4$-Alkyl-tert.-butyläther abzieht, das Bodenprodukt danach einer zweiten, einen sauren Katalysator enthaltenden Reaktionszone (11) zuführt, in der der $C_3$- oder $C_4$-Alkyl-tert.-butyläther bei erhöhter Temperatur in Isobuten und primären $C_3$- oder $C_4$-Alkohol zerlegt wird, das Gemisch aus Isobuten und primären $C_3$- oder $C_4$-Alkohol einer zweiten Destillationszone (13) zuführt, in der als Kopfprodukt Isobuten ohne Einschaltung einer Wasserwäsche mit einem Gehalt an primärem $C_3$- oder $C_4$-Alkohol von höchstens 500 Gewichts-ppm und als Bodenprodukt der primäre $C_3$- oder $C_4$-Alkohol abgezogen werden, und den erhaltenen primären $C_3$- oder $C_4$-Alkohol in die Reaktionszone (4) für die Verätherung zurückführt.

Verfahren zur Gewinnung von Isobuten aus Isobuten enthaltenden $C_4$-Kohlenwasserstoffgemischen

Die vorliegende Erfindung betrifft ein Verfahren zur Gewinnung von Isobuten aus Isobuten enthaltenden $C_4$-Kohlenwasserstoffgemischen durch Umsetzung des Gemisches mit einem primären $C_3$- oder $C_4$-Alkohol, Abtrennung des gebildeten tertiären Äthers und Zerlegung desselben bei erhöhten Temperaturen.

Es ist bereits bekannt, Isobuten durch Anwendung von Schwefelsäure-Extraktionsverfahren aus $C_4$-Kohlenwasserstoffgemischen zu gewinnen. Bei diesen Schwefelsäure-Extraktionsverfahren muß Schwefelsäure hoher Konzentration verwendet werden, und infolgedessen ist die Verwendung kostspieliger Materialien für die Ausrüstung erforderlich. Da weiterhin Nebenreaktionen des Isobutens, beispielsweise Dimerisation, Polymerisation, Hydratation und dergl., während der Extraktion erfolgen, ist das Schwefelsäure-Extraktionsverfahren hinsichtlich Ausbeute und Qualität der Produkte nicht zufriedenstellend.

Es ist weiter, z.B. aus der DE-PS 12 16 865, DE-AS 19 34 422 oder DE-AS 20 11 826, ein Verfahren zur Gewinnung von Isobuten bekannt, bei dem in einer ersten Stufe ein Isobuten enthaltendes $C_4$-Kohlenwasserstoffge-

Ste/EL

misch mit Methanol umgesetzt wird und der gebildete Methyl-tert.-butyläther in einer zweiten Stufe in Methanol und Isobuten zerlegt wird. Die bekannten Verfahren haben jedoch den Nachteil, daß Methanol mit den $C_4$-Kohlenwasserstoffen azeotrope Gemische bildet. So ist beispielsweise aus der DE-OS 26 29 769 und der DE-AS 19 34 422 bekannt, daß bei der Herstellung des Methyl-tert.-butyläthers bei der destillativen Abtrennung der nicht umgesetzten Kohlenwasserstoffe aus dem Reaktionsgemisch die abgetrennten Kohlenwasserstoffe aufgrund von Kohlenwasserstoff/Methanol-Azeotropen etwa 2 % Methanol enthalten, das nur durch aufwendige Verfahren, z.B. durch Einschaltung einer Wasserwäsche, zurückgewonnen werden kann. Besonders nachteilig wirkt sich aus, daß auch bei der destillativen Auftrennung des aus Isobuten und Methanol bestehenden Reaktionsgemisches der Zerlegungsstufe Methanol und Isobuten ein azeotropes Gemisch bilden, so daß auch in der Zerlegungsstufe eine aufwendige Wasserwäsche eingeschaltet werden muß (vgl. z.B. DE-AS 19 34 422), um die Methanol-Verluste niedrig zu halten und ein Methanol-freies Isobuten, wie es für die meisten Anwendungsfälle erforderlich ist, zu erhalten.

Neben der Verwendung von Methanol und allenfalls Äthanol als Alkohole für die Verätherungsreaktion sind zwar bereits allgemein primäre Alkohole als mögliche Reaktionspartner für die Umsetzung zum tertiären Äther genannt worden (vgl. z.B. die bereits vorstehend genannten DE-PS 12 16 865, DE-AS 19 34 422 oder DE-AS 20 11 826). Gegen die Verwendung höherer primärer Alkohole, z.B. von $C_3$- oder $C_4$-Alkoholen, bestand jedoch ein erhebliches Vorurteil, da bekannt war, daß solche höheren primären Alkohole unter den Reaktionsbedingungen der Zerlegungsstufe in Gegenwart eines sauren Katalysators leicht zu Olefinen dehydratisiert werden können. So wird z.B. in der

0022510

bereits genannten DE-AS 19 34 422, Spalte 3, 1. Absatz ausdrücklich darauf hingewiesen, Methanol, das sich nicht dehydratisieren läßt, als Alkohol anzuwenden, um die unerwünschte Bildung von Olefinen in der Zerlegungsstufe zu vermeiden.

Ein weiteres erhebliches Vorurteil gegen die Verwendung höherer primärer Alkohole ergab sich daraus, daß bekannt war, z.B. aus der US-PS 3 170 000, inbesondere Tabelle I und Spalte 3, Zeilen 39 bis 31, daß Methanol und Äthanol bei der Verätherungsreaktion wesentlich höhere Ausbeuten ergeben als die höheren primären Alkohole, z.B. die $C_3$- oder $C_4$-Alkohole.

Wegen der vorstehend beschriebenen Nachteile und Vorurteile haben die bekannten Verfahren zur Isobutengewinnung unter Zerlegung des in einer ersten Verätherungsstufe erhaltenen tertiären Äthers keine großtechnische Anwendung gefunden, sondern sind nur papierner Stand der Technik geblieben, und man war daher für die großtechnische Gewinnung von Isobuten auf die Anwendung von Schwefelsäure-Extraktionsverfahren mit den diesen Verfahren anhaftenden Mängeln und Nachteilen angewiesen.

Der Erfindung lag daher die Aufgabe zugrunde, ein Verfahren zur Gewinnung von Isobuten aus Isobuten enthaltenden $C_4$-Kohlenwasserstoffen zur Verfügung zu stellen, welches die Nachteile der bekannten Verfahren nicht aufweist.

Es wurde nun ein einfaches Verfahren gefunden, zu Gewinnung von Isobuten aus Isobuten enthaltenden $C_4$-Kohlenwasserstoffgemischen durch Umsetzung des Gemisches mit einem primären Alkohol in Gegenwart eines sauren Kondensationsmittels und Zerlegung des gebildeten tertiären Äthers in Gegenwart eines sauren Katalysators bei erhöhten Tempera- turen, welches dadurch gekennzeichnet ist, daß man als primären Alkohol einen primären $C_3$- oder $C_4$-Alkohol und als saures Kondensationsmittel für die Ätherbildung Ionenaustauscher in der Wasserstofform verwendet, wobei man den primären $C_3$- oder $C_4$-Alkohol und das $C_4$-Kohlenwasserstoffgemisch, gegebenenfalls nach vorheriger Vermischung, zunächst der Reaktionszone für die Verätherung, in der sich der Ionenaustauscher befindet, zuführt, das aus der Reaktionszone für die Verätherung erhaltene Reaktionsgemisch anschließend in einer ersten Destillationszone destilliert, als Kopfprodukt ohne Einschaltung einer Wasserwäsche ein die nicht umgesetzten Kohlenwasserstoffe enthaltendes $C_4$-Kohlenwasserstoffgemisch mit einem Gehalt an primären $C_3$- oder $C_4$-Alkohol von höchstens 1000 Gewichts-ppm und als Bodenprodukt den gebildeten, gegebenenfalls noch im Überschuß zugesetzten primären $C_3$- oder $C_4$-Alkohol enthaltenden $C_3$- oder $C_4$-Alkyl-tert.-butyläther abzieht, das Bodenprodukt danach einer zweiten, einen sauren Katalysator enthaltenden Reaktionszone zuführt, in der der $C_3$- oder $C_4$-Alkyl-tert.-butyläther bei erhöhter Temperatur in Isobuten und primären $C_3$- oder $C_4$-Alkohol zerlegt wird, das Gemisch aus Isobuten und primären $C_3$- oder $C_4$-Alkohol einer zweiten Destillationszone zuführt, in der als Kopfprodukt Isobuten ohne Einschaltung einer Wasserwäsche mit einem Gehalt an primärem $C_3$- oder $C_4$-Alkohol von höchstens 500 Gewichts-ppm und als Bodenprodukt der primäre $C_3$- oder $C_4$-Alkohol abgezogen werden, und den erhaltenen primären $C_3$- oder $C_4$-Alkohol in die Reaktionszone für die Verätherung zurückführt.

Nach dem neuen Verfahren wird aus dem nach der Verätherungsstufe erhaltenen Reaktionsgemisch ein praktisch $C_3$- oder $C_4$-Alkohol-freies $C_4$-Kohlenwasserstoff-Raffinat durch einfache Destillation ohne Einschaltung einer Wasserwäsche abgetrennt, da nicht umgesetzter primärer $C_3$- oder $C_4$-Alko-

0022510

hol überraschenderweise keine Azeotrope mit den $C_4$-Kohlenwasserstoffen bildet. Im allgemeinen wird als Kopfprodukt der Destillation ein $C_4$-Kohlenwasserstoff-Raffinat mit einem Gehalt an $C_3$- oder $C_4$-Alkohol von höchstens 1000 Gewichts-ppm, vorzugsweise höchstens 500 Gewichts-ppm, insbesondere höchstens 100 Gewichts-ppm abgezogen. Auch bei der destillativen Trennung des bei der Zerlegung des $C_3$- oder $C_4$-Alkyl-tert.-butyläthers erhaltenen Reaktionsgemisches in Isobuten und den $C_3$- oder $C_4$-Alkohol werden keine Azeotrope des Alkohols gebildet. Der $C_3$- oder $C_4$-Alkohol kann daher in einfacher Weise ohne Einschaltung einer Wasserwäsche praktisch ohne Verluste zurückgewonnen und in die Verätherungsstufe zurückgeführt werden.

Überraschenderweise wird nach dem erfindungsgemäßen Verfahren Isobuten in hoher Ausbeute, z.B. in einer Ausbeute von mehr als 97 %, bezogen auf das im eingesetzten $C_4$-Kohlenwasserstoffgemisch enthaltene Isobuten, erhalten. Dies war unerwartet, da in der bereits vorstehend genannten US-PS 3 170 000, Spalte 3, beschrieben ist, daß bei der Verwendung von $C_3$- oder $C_4$-Alkoholen nur sehr schlechte Ausbeuten an tertiärem Äther erhalten werden. Auch aus der US-PS 3 634 535, besonders Spalte 6, ist bekannt, daß die Umsetzung von Isobuten mit Propanol nur in einer Ausbeute von etwa 50 % zur Bildung des tertiären Äthers führt, während bei der entsprechenden Umsetzung von Isobuten und Methanol Ausbeuten von etwa 90 bis 95 % erhalten werden. Es war daher überraschend, daß nach dem erfindungsgemäßen Verfahren Ausbeuten an tertiärem Äther von mehr als 95 % erhalten werden.

Für das erfindungsgemäße Verfahren geeignete Isobuten enthaltende $C_4$-Kohlenwasserstoffgemische werden beispelsweise beim thermischen oder katalytischen Cracken von Erdölprodukten, bei der Herstellung von Äthylen durch Pyro-

0022510

lyse von verflüssigtem Petroleumgas (LPG), Leichtbenzin (Naphtha), Gasöl oder dergl. oder bei der katalytischen Dehydrierung von n-Butan und/oder n-Buten erhalten. Diese $C_4$-Kohlenwasserstoffgemische enthalten in der Regel neben dem Isobuten olefinische und paraffinische $C_4$-Kohlenwasserstoffe und können darüber hinaus noch Butadien, z.B. in einer Menge von bis zu 70 Gewichtsprozent, und höhere Acetylene, wie Butin-1 und Butenin enthalten. Butadien enthaltende $C_4$-Kohlenwasserstoffgemische können als solche oder nach vorheriger Abtrennung des Butadiens aus dem $C_4$--Kohlenwasserstoffgemisch, z.B. durch Butadien-Extraktion unter Verwendung eines selektiven Lösungsmittels, verwendet werden. Die $C_4$-Kohlenwasserstoffgemische können außerdem noch $C_3$-Kohlenwasserstoffe wie Propan, Propen, Propin z.B. bis zu 10 Gewichtsprozent, enthalten. Die $C_4$-Kohlenwasserstoffgemische weisen im allgemeinen einen Isobutengehalt von 5 bis 95 Gewichtsprozent, vorzugsweise 10 bis 90 Gewichtsprozent, insbesondere 20 bis 70 Gewichtsprozent, auf. Vorzugsweise werden solche $C_4$-Kohlenwasserstoffgemische verwendet, die neben Isobuten, n-Butan, Isobutan, Buten-1, trans-Buten-2 und cis-Buten-2 und gegebenenfalls Butadien-1,3 enthalten.

Als erfindungsgemäß zu verwendende primäre $C_3$- oder $C_4$--Alkohole (d.h. Alkohole mit 3 oder 4 Kohlenstoffatomen) werden im allgemeinen n-Propanol, n-Butanol oder Isobutanol, vorzugsweise n-Propanol oder Isobutanol, und insbesondere Isobutanol eingesetzt. Die Alkohole werden z.B. als technische Produkte üblicher Reinheit, beispielsweise mit einer Reinheit von mindestens 95 %, vorzugsweise mindestens 98 % verwendet.

Als saure Kondensationsmittel für die Verätherung, die die erste Stufe darstellt, werden Ionenaustauscher in der Wasserstoffform verwendet. Geeignete Ionenaustauscher sind

0022510

beispielsweise sulfonierte Kohlen, sulfonierte Phenol-Formaldehyd-Harze, sulfonierte von Cumaron-Inden-Kondensationsprodukten abgeleitete Harze sowie insbesondere sulfonierte
Polystyrol-Harze wie Kern-sulfonierte vernetzte Styrol-Divinylbenzol-Copolymerisate. Die Menge des Ionenaustauschers beträgt im allgemeinen 0,01 bis 1 l Schüttvolumen
pro 1 Reaktorvolumen. Die Ionenaustauscher können als
solche oder auf einem Trägermaterial verwendet werden.
Geeignete Trägermaterialien sind z.B. Aluminiumoxid,
Kieselsäure, Aktivkohle, Kunststoffe wie Styrol-Polymerisate. Für die Verätherung können z.B. Rührkessel- oder
Festbettreaktoren verwendet werden. Vorzugsweise werden
Festbettreaktoren eingesetzt.

Mit besonderem Vorteil wird die Verätherung in der Weise
durchgeführt, daß die Austrittstemperatur des Reaktionsgemisches aus der Reaktionszone für die Verätherung im Bereich von 25 bis 65$^{o}$C, vorzugsweise 30 bis 60$^{o}$C, insbesondere 30 bis 50$^{o}$C liegt. Vorzugsweise werden Austrittstemperaturen angewendet, die tiefer liegen, als die mittlere
Temperatur in der Verätherungsstufe. Im allgemeinen wird
in der Verätherungsreaktion das im $C_4$-Kohlenwasserstoffge-
misch enthaltene Isobuten zu mindestens 90 %, vorzugsweise
zu mindestens 95 %, insbesondere zu mindestens 96 % zum
$C_3$- oder $C_4$-Alkyl-tert.-butyläther umgesetzt.

Die erfindungsgemäße Verätherung kann bei Normaldruck erfolgen. Es ist jedoch zweckmäßig, einen geringen Überdruck
z.B. Drücke von 1,01 bis 30 bar, insbesondere 2 bis
20 bar, anzuwenden. Das Isobuten enthaltende $C_4$-Kohlenwas-
serstoffgemisch kann dabei je nach Druck und Temperatur
für die Umsetzung flüssig oder gasförmig eingesetzt werden. Vorzugsweise werden flüssige Isobuten enthaltende
$C_4$-Kohlenwasserstoffgemische eingesetzt. Die Verätherung
kann diskontinuierlich durchgeführt werden. Die Reaktions- zeiten liegen bei diskontinuierlicher Arbeitsweise im allgemeinen zwischen 1 Minute und 5 Stunden. Vorzugsweise wird die Verätherung jedoch kontinuierlich durchgeführt, wobei das Verhältnis aus dem Volumen der Reaktionszone (in Volumeneinheiten) und dem Durchsatz in Volumeneinheiten je Stunde im allgemeinen 0,01 bis 5 Stunden, vorzugsweise 0,02 bis 1 Stunde, insbesondere 0,03 bis 1 Stunde, beträgt.

Das Gewichtsverhältnis von primären $C_3$- oder $C_4$-Alkohol zu dem im $C_4$-Kohlenwasserstoffgemisch enthaltenen Isobuten beträgt bei der Verätherung im allgemeinen 100 : 1 bis 1 : 1, vorzugsweise 20 : 1 bis 1,2 : 1, insbesondere 4 : 1 bis 1,3 : 1.

Das aus der Reaktionszone für die Verätherung erhaltene Reaktionsgemisch, welches in der Regel noch im Überschuß zur Verätherung zugesetzten primären $C_3$- oder $C_4$-Alkohol enthält, wird durch Destillation ohne Einschaltung einer Wasserwäsche aufgetrennt, wobei als Kopfprodukt ein weitgehend isobutenfreies $C_4$-Kohlenwasserstoffgemisch-Raffinat abgezogen wird, welches im allgemeinen einen Isobuten-Gehalt von höchstens 5 Gewichtsprozent, vorzugsweise höchstens 2,5 Gewichtsprozent, insbesondere höchstens 1,5 Gewichtsprozent aufweist. Vorzugsweise wird als Kopfprodukt ein $C_4$-Kohlenwasserstoff-Raffinat mit einem Gehalt von höchstens 200 Gewichts-ppm $C_3$- oder $C_4$-Alkyl-tert.-butyläther und/oder Di-$C_3$-alkyl- oder Di-$C_4$-alkyläther abgezogen.

Als Bodenprodukt der Destillation des nach der Verätherung erhaltenen Reaktionsgemisches wird der gegebenenfalls noch überschüssigen primären $C_3$- oder $C_4$-Alkohol enthaltende $C_3$- oder $C_4$-Alkyl-tert.-butyläther erhalten. Zweckmäßig wird ein Bodenprodukt mit einem Gehalt an $C_4$-Kohlenwasser-

stoffen von höchstens 1000 Gewichts-ppm, vorzugsweise höchstens 500 Gewichts-ppm, insbesondere höchstens 100 Gewichts-ppm, abgezogen.

Der erhaltene tertiäre Äther wird anschließend in der zweiten Stufe des Verfahrens in Gegenwart eines sauren Katalysators bei erhöhten Temperaturen in Isobuten und primären $C_3$- oder $C_4$-Alkohol zerlegt. Als Ausgangsprodukt für die Zerlegung kann tertiärer Äther, der praktisch frei von $C_3$- oder $C_4$-Alkohol ist, verwendet werden, der beispielsweise durch Verwendung einer Menge an $C_3$- oder $C_4$-Alkohol bei der Verätherung, die höchstens der stöchiometrisch erforderlichen Alkoholmenge entspricht, oder durch Abtrennung, z.B. durch Abdestillieren, von überschüssig zugesetztem primärem $C_3$- oder $C_4$-Alkohol aus dem nach der Destillation des Reaktionsgemisches der Verätherung erhaltenen Bodenprodukt erhalten worden ist. Vorzugsweise wird der nach der destillativen Abtrennung des $C_4$-Kohlenwasserstoffgemisch-Raffinats als Bodenprodukt erhaltene tertiäre Äther ohne weitere Abtrennung von gegebenenfalls vorhandenem überschüssigem $C_3$- oder $C_4$-Alkohol für die Zerlegung eingesetzt. Es ist jedoch auch möglich, nur einen Teil des überschüssigen $C_3$- oder $C_4$-Alkohols abzutrennen. Im allgemeinen wird der gebildete $C_3$- oder $C_4$-Alkyl-tert.-butyläther ohne Wasserzusatz in der Zerlegungsstufe zerlegt.

Für die Zerlegung wird der tertiäre Äther verdampft und mit dem sauren Katalysator in der Dampfphase kontaktiert. Als saure Katalysatoren kommen beispielsweise Ionenaustauscher in der Wasserstofform, wie sulfonierte Kohlen, sulfonierte Phenol-Formaldehydharze, sulfonierte von Cumaron-Inden-Kondensationsprodukten abgeleitete Harze sowie insbesondere sulfonierte Polystyrolharze wie Kern-sulfonierte, vernetzte Styrol-Divinylbenzol-Copolymerisate in Betracht.

Weiter sind feste Phosphorsäurekatalysatoren, die Mono- oder vorzugsweise Polyphosphorsäure auf einem festen Trägermaterial enthalten, vorteilhaft geeignet. Geeignete Trägermaterialien für die Phosphorsäurekatalysatoren sind z.B. Aluminiumoxid, Kieselsäure, Aktivkohle, Kieselgur oder Bims. Vorzugsweise wird Kieselgel als Trägermaterial verwendet.

Weitere geeignete saure Katalysatoren sind saure Metallsulfate wie Natriumhydrogensulfat, Calciumhydrogensulfat, Aluminiumsulfate, Nickelsulfate, Kupfersulfat, Kobaltsulfat, Cadmiumsulfat, Strontiumsulfat. Diese sauren Metallsulfate können als solche verwendet werden. Vorzugsweise werden sie auf einem Trägermaterial angewendet. Geeignete Trägermaterialien sind z.B. Kieselgel, Aktivkohle, Aluminiumoxid oder Bims.

Weiter kommen Kieselgel oder Aluminiumoxid alleine als Katalysatoren für die Zerlegung in Betracht.

In einer weiteren Ausführungsform des erfindungsgemäßen Verfahrens wird für die Zerlegung als saurer Katalysator ein Metallphosphat, insbesondere ein Metallhydrogenphosphat, verwendet. Diese Phosphate können Phosphorsäure auch im Überschuß, der über die stöchiometrische Zusammensetzung der sauren Metallphosphate hinausgeht, enthalten, z.B. in einem Überschuß bis zu 65 %, vorzugsweise 1 bis 50 %, insbesondere 10 bis 20 %. Als derartige Metallphosphate können beispielsweise Magnesiumphosphate, Calciumphosphate, Strontiumphosphate, Bariumphosphate, Manganphosphate, Nickelphosphate, Kupferphosphate, Kobaltphosphate, Cadmiumphosphate, Eisen(II)-phosphate, Chromphosphate und insbesondere Aluminiumphosphate verwendet werden. Der Metallphosphat-Katalysator kann als solcher oder auf einem Trägermaterial verwendet werden. Geeignete Trägermateria-

0022510

lien sind beispielsweise Aluminiumoxid, Kieselsäure, Aktivkohle, Zinkoxid.

Die Menge des sauren Katalysators beträgt im allgemeinen etwa 0,01 bis 1 kg, vorzugsweise etwa 0,03 bis 0,3 kg je 1 kg/h Durchsatz des teritären Äthers durch den Reaktor. Vorzugsweise werden für die Zerlegung des tertiären Äthers Festbettreaktoren verwendet.

Die Zerlegungstemperatur des tertiären Äthers variiert in Abhängigkeit von der Art des sauren Katalysators und der Kontaktzeit, liegt jedoch im allgemeinen bei Temperaturen von 50 bis 350°C, vorzugsweise 80 bis 300°C, insbesondere 100 bis 250°C. Bei der Verwendung von Metallphosphaten oder Phosphorsäurekatalysatoren als Zerlegungskatalysatoren werden im allgemeinen Temperaturen von 80 bis 350°C, vorzugsweise 90 bis 260°C, insbesondere 170 bis 210°C angewendet.

Die Kontaktzeit des verdampften tertiären Äthers beträgt zweckmäßig 0,1 bis 20 Sekunden, vorzugsweise 1 bis 10 Sekunden.

Die Zerlegung des tertiären Äthers kann bei Normaldruck erfolgen. Im allgemeinen wird jedoch Überdruck angewendet, z.B. Drücke bis zu 30 bar, vorzugsweise bis zu 20 bar. Vorteilhaft wird die Zerlegung des tertiären Äthers bei Drücken von 2 bis 15 bar, vorzugsweise 3 bis 12 bar, insbesondere 4 bis 12 bar ausgeführt. Die Zerlegung kann jedoch auch bei vermindertem Druck durchgeführt werden.

Die Zerlegung des tertiären Äthers kann diskontinuierlich erfolgen. Vorzugsweise wird sie jedoch kontinuierlich durchgeführt.

Das bei der Zerlegung erhaltene Reaktionsgemisch, welches als Reaktionsprodukte Isobuten und primären $C_3$- oder $C_4$-Alkohol enthält, wird einer zweiten Destillationszone zugeführt, in der als Kopfprodukt Isobuten ohne Einschaltung einer Wasserwäsche mit einem Gehalt an primärem $C_3$- oder $C_4$-Alkohol von höchstens 500, vorzugsweise höchstens 100, insbesondere höchstens 50 Gewichts-ppm abgezogen wird. Zweckmäßig wird ein Kopfprodukt mit einer Reinheit von mindestens 99,3 Gew.%, vorzugsweise mindestens 99,5 Gew.%, insbesondere mindestens 99,7 Gew.%, und einem Gehalt an dem gegebenenfalls in sehr kleinen Mengen als Nebenprodukt gebildeten Di-$C_3$-alkyl- oder Di-$C_4$-alkyläther und/oder $C_3$- oder $C_4$-Alkyl-tert.-butyläther von höchstens 100 Gewichts-ppm, vorzugsweise höchstens 50 Gewichts-ppm, insbesondere höchstens 20 Gewichts-ppm abgezogen. Der aus der zweiten Destillationszone als Bodenprodukt erhaltene primäre $C_3$- oder $C_4$-Alkohol wird in die Reaktionszone für die Verätherung zurückgeführt. Vorzugsweise wird der Gehalt des zurückgeführten primären $C_3$- oder $C_4$-Alkohols an gegebenenfalls in sehr kleinen Mengen als Nebenprodukt gebildetem und sich im zurückgeführten primären $C_3$- oder $C_4$-Alkohol anreicherndem Di-$C_3$-Alkyl- oder Di-$C_4$-Alkyläther auf 2 bis 20 Gew.% begrenzt.

Bei dem neuen Verfahren kann es zweckmäßig sein, bei der Verwendung von Isobutanol als $C_4$-Alkohol aus dem Isobutanolstrom zur Entfernung von eventuell angereicherten Verunreinigungen einen Isobutanolteilstrom abzuzweigen, wobei die Abzweigung zweckmäßig als Seitenabzug aus der zweiten Destillationszone oder aus dem aus der zweiten Destillationszone abgezogenen Bodenprodukt erfolgt. Zweckmäßig wird aus dem Isobutanolstrom ein Teilstrom abgezweigt, der im allgemeinen 0,1 bis 10 Gew.%, vorzugsweise 0,5 bis 5 Gew.% des Isobutanolstromes beträgt. Vorteilhaft wird zur Ausschleusung von gegebenenfalls gebildetem Diisobutyl-

0022510

äther ein 3 bis 40 Gew.%, vorzugsweise 5 bis 35 Gew.%, insbesondere 10 bis 30 Gew.% Diisobutyläther enthaltender Isobutanol-Teilstrom abgezweigt.

In einer vorteilhaften Ausführungsform des Verfahrens wird der Isobutanolteilstrom in an sich bekannter Weise in Gegenwart eines Dehydratisierungskatalysators dehydratisiert, wobei außer dem Isobutanol auch der Diisobutyläther dehydratisiert wird, wodurch die Ausbeute an Isobuten zusätzlich erhöht wird.

Zweckmäßig wird die Dehydratisierung in der Gasphase an einem Katalysator durchgeführt. Geeignete Katalysatoren sind z.B. Kieselgel, Thoriumoxid, Titan(IV)-oxid und insbesondere Aluminiumoxid. Im allgemeinen werden für die Dehydratisierung Temperaturen von 250 bis 450°C, vorzugsweise 300 bis 400°C angewendet. Es kann vorteilhaft sein, die Dehydratisierung in Gegenwart von gegebenenfalls zugesetztem Wasser durchzuführen.

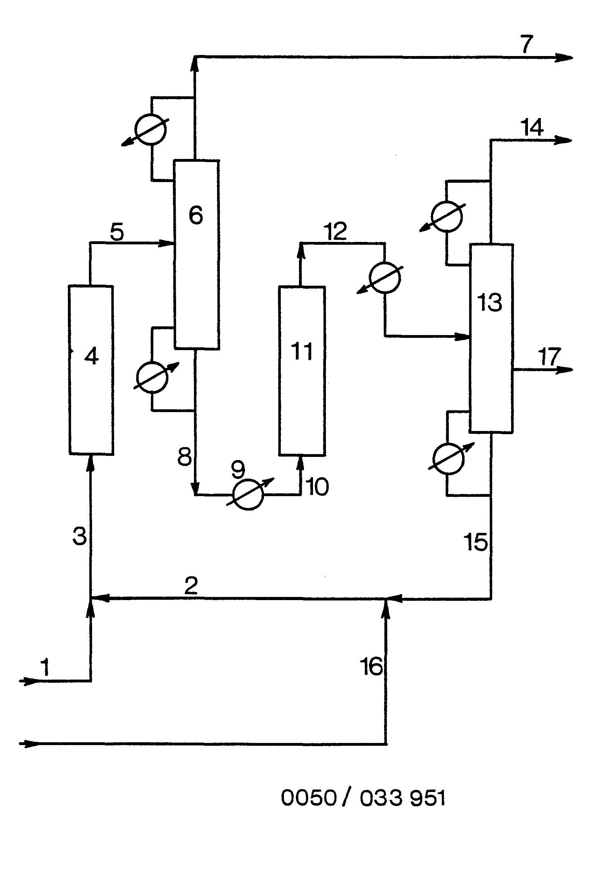

In der Figur wird eine beispielhafte Ausführungsform des erfindungsgemäßen Verfahrens schematisch erläutert. Das Isobuten enthaltende $C_4$-Kohlenwasserstoffgemisch (durch Leitung 1) und der primäre $C_3$- oder $C_4$-Alkohol (durch Leitung 2) werden vermischt, und die erhaltene Mischung wird durch Leitung 3 dem Reaktor 4 für die Verätherung zugeführt, in dem sich der Ionenaustauscher befindet. Zweckmäßig ist der Reaktor als Festbettreaktor, z.B. als Strömungsrohr oder Schlaufenreaktor oder Kombination der beiden Reaktortypen ausgestaltet. Es kann jedoch auch ein anderer Reaktor-Typ, z.B. ein Rührkessel oder eine Rührkesselkaskade, verwendet werden. Das erhaltene Reaktionsgemisch wird aus dem Reaktor über Leitung 5 abgezogen und einer ersten Destillationskolonne 6 zugeführt. Am Kopf der Destillationskolonne wird weitgehend isobutenfreies $C_4$-Koh-

lenwasserstoffgemisch (Raffinat) durch Leitung 7 abgezogen. Der als Bodenprodukt der Destillationskolonne 6 erhaltene tertiäre Äther, der gegebenenfalls noch im Überschuß zugesetzten primären $C_3$- oder $C_4$-Alkohol enthält, wird über Leitung 8 zunächst dem Verdampfer 9 zugeführt und nach der Verdampfung über Leitung 10 in den Reaktor 11 eingeleitet, in dem sich der saure Katalysator befindet. Reaktor 11 ist im allgemeinen ein Festbett-Reaktor. Das aus Reaktor 11 abgezogene Gemisch aus Isobuten und primärem $C_3$- oder $C_4$-Alkohol wird über Leitung 12 in die Destillationskolonne 13 geleitet, in der als Kopfprodukt hochreines Isobuten erhalten wird, welches über Leitung 14 abgezogen wird. Der als Bodenprodukt erhaltene $C_3$- oder $C_4$-Alkohol wird über Leitung 15 und 2, gegebenenfalls nach ergänzender Zugabe von $C_3$- oder $C_4$-Alkohol über Leitung 16, dem Reaktor 4 für die Verätherung wieder zugeführt. Über Leitung 17 wird zweckmäßig ein kleiner $C_3$- oder $C_4$-Alkohol enthaltender Teilstrom zur Ausschleusung von eventuell gebildeten Verunreinigungen wie Diisobutyläther, Diisobuten, Triisobuten, abgezogen. Dieser Teilstrom kann bei der Verwendung von Isobutanol als $C_3$- oder $C_4$-Alkohol einem Dehydratisierungsreaktor zugeführt werden, in dem zusätzlich Isobuten erhalten wird.

Nach dem erfindungsgemäßen Verfahren wird ein hochreines Isobuten erhalten, welches insbesondere für die Herstellung von hochmolekularen Polymeren des Isobutens geeignet ist.

Die folgenden Beispiele veranschaulichen die Erfindung.

Beispiel 1

Die Verätherung wurde unter Verwendung eines $C_4$-Kohlenwasserstoffgemisches durchgeführt, welches den Rest (Raffi- nat) einer aus einer Äthylenanlage erhaltenen $C_4$-Fraktion darstellte, aus der das Butadien extrahiert worden war. Die Zusammensetzung des $C_4$-Kohlenwasserstoffgemisches nach der Butadien-Extraktion war wie folgt:

| | |
|---|---|
| Isobutan | 1,9 Vol.% |
| n-Butan | 8,1 Vol.% |
| Isobuten | 46,0 Vol.% |
| Buten-1 | 26,7 Vol.% |
| trans-Buten-2 | 10,1 Vol.% |
| cis-Buten-2 | 7,0 Vol.% |
| Butadien-1,3 | 0,2 Vol.% |

Eine Mischung von 258 g je Stunde dieses $C_4$-Kohlenwasserstoffgemisches mit 320 ml je Stunde Isobutanol wurde in ein rohrförmiges Reaktionsgefäß aus rostfreiem Stahl eingeleitet, in dem sich 254 ml eines sulfonierten Polystyroldivinylbenzol-harzes in der Wasserstofform (Lewatit SPC 118, Kornfraktion 0,8 bis 1 mm) befanden. In dem Reaktionsgefäß wurde eine Temperatur von 40°C und ein Druck von 12 bar aufrechterhalten. Das erhaltene Reaktionsgemisch wurde einer Destillationskolonne zugeführt, wobei am Kopf der Kolonne ein Buten-/Butan-Raffinat mit einem Isobutengehalt von weniger als 2 Gewichtsprozent erhalten wurde. Das Raffinat war praktisch Isobutanol-frei und konnte daher ohne weitere Reinigungsoperationen, z.B. ohne Einschaltung einer Wasserwäsche, unmittelbar als Ausgangsstoff für weitere Reaktionen verwendet werden. Am Sumpf der Destillationskolonne wurden 500 ml je Stunde Isobutyl--tert.-butyläther, der noch 24,3 Gewichtsprozent, bezogen auf das Gemisch, im Überschuß zugesetztes Isobutanol enthielt, abgezogen und in einen Verdampfer eingeleitet. Der verdampfte, auf 190°C erhitzte Isobutyl-tert.-butyläther wurde zur Ätherspaltung in einen rohrförmigen Spaltreaktor, welcher als Spaltkatalysator Phosphorsäure auf

Kieselgel (20 % Phosphorsäureüberschuß) enthielt, eingeleitet und bei 190°C in Isobuten und Isobutanol aufgespalten. Das Reaktionsprodukt der Ätherspaltung wurde in eine zweite Destillationskolonne eingeleitet, in der am Kopf 115 g je Stunde hochreines Isobuten der folgenden Zusammensetzung erhalten wurde:

| | | |
|---|---|---|
| Isobuten | 99,85 | Gew.% |
| Isobutan | 730 | Gew. ppm |
| Butan | 3 | Gew. ppm |
| Buten-1 | 420 | Gew. ppm |
| trans-Buten-2 | 190 | Gew. ppm |
| cis-Buten-2 | 160 | Gew. ppm |
| Butadien-1,3 | 19 | Gew. ppm |

Die Ausbeute an Isobuten bezogen auf das im eingesetzten $C_4$-Kohlenwasserstoffgemisch enthaltene Isobuten betrug 97,7 %. Am Sumpf der zweiten Destillationskolonne wurden 320 ml je Stunde Isobutanol erhalten, welches wieder in die Verätherungsreaktion rückgeführt wurde.

Die Durchsätze an $C_4$-Kohlenwasserstoffgemisch und Isobutanol durch den Reaktor für die Verätherungsreaktion konnten um den Faktor 4 erhöht werden bei praktisch unveränderten Reinheiten des bei der Destillation erhaltenen Buten-/Butan-Raffinates und des am Boden der Destillationskolonne erhaltenen, den Isobutyl-tert.-butyläther enthaltenden Produktes.

Vergleichsbeispiel 1

Die Verätherung wurde wie in Beispiel 1 beschrieben bei 40°C durchgeführt, wobei jedoch anstelle des Isobutanols die entsprechende stöchiometrische Menge Methanol eingesetzt wurde. Bei einem Durchsatz des Einsatzgemisches von

0022510

2 l/h je 1 l Reaktorvolumen betrug der Restgehalt an Isobuten in dem nach der Destillation erhaltenen Buten-/Butan-Raffinat noch mehr als 30 Gewichtsprozent. Das Buten-/Butan-Raffinat enthielt außerdem mehr als 1,5 Mol.% Methanol, welches durch eine Wasserbehandlung aus dem Raffinat ausgewaschen wurde. Aus dem nach der Wasserwäsche erhaltenen Methanol-Wassergemisch wurde das Methanol zurückgewonnen und in die Verätherungsreaktion zurückgeführt. Das Buten/Butan-Raffinat wurde anschließend einer Trocknung unterzogen, um das eingeschleppte Wasser zu entfernen.

Dagegen wird bei der Verwendung von Isobutanol (gemäß Beispiel 1) anstelle von Methanol durch einfache Destillation ein Buten-/Butan-Raffinat mit einem Isobutanol-Gehalt von weniger als 1 ppm erhalten.

Vergleichsbeispiel 2

Die Ätherspaltung wurde wie in Beispiel 1 beschrieben durchgeführt, wobei jedoch anstelle des Isobutyl-tert.-butyläthers der Methyl-tert.-butyläther verwendet wurde. Am Kopf der Destillationskolonne wurde Isobuten der folgenden Zusammensetzung erhalten:

| | |
|---|---|
| Isobuten | 97,0 Gew.% |
| Andere Kohlenwasserstoffe | 0,15 Gew.% |
| Methanol | 2,45 Gew.% |
| Dimethyläther | 0,4 Gew.% |

Das erhaltene Isobuten weist einen Methanol-Gehalt von 2,45 Gew.% und daneben einen Dimethyläther-Gehalt von 0,4 Gew.% auf, während im gemäß Beispiel 1 erhaltenen Isobuten der Gehalt an Isobutanol und an Diisobutyläther unterhalb der Nachweisbarkeitsgrenze ( 4 ppm) liegt.

0022510

Das im Vergleichsbeispiel erhaltene Isobuten muß für viele Verwendungszwecke, z.B. bei der Verwendung als Ausgangsstoff für die kationische Polymerisation mit Borfluorid, weiteren Reinigungsoperationen unterworfen werden, während das gemäß Beispiel 1 erhaltene hochreine Isobuten unmittelbar verwendet werden kann. Um aus dem nach Vergleichsbeispiel 2 erhaltenen Isobuten ein Isobuten mit vergleichbarer Reinheit wie der des Isobuten nach Beispiel 1 zu erhalten wären beispielsweise die folgenden zusätzlichen Verfahrensschritte erforderlich:

1.  Destillation des Isobutens zur Dimethyläther-Abtrennung

2.  Anschließende Extraktion des Isobutens mit Wasser zur Entfernung des Methanols

3.  Trocknung des erhaltenen wasserhaltigen Isobutens

4.  Destillation des aus der Extraktion erhaltenen Methanol-Wasser-Gemisches zur Rückgewinnung des Methanols.

## Beispiel 2

Die Verätherung wurde unter Verwendung des $C_4$-Schnittes einer Äthylenanlage durchgeführt. Die Zusammensetzung des $C_4$-Kohlenwasserstoffgemisches war wie folgt:

| | |
|---|---|
| Butan | 3,65 Gew.% |
| Isobutan | 1,41 Gew.% |
| Buten-1 | 20,44 Gew.% |
| Isobuten | 23,52 Gew.% |
| trans-Buten-2 | 4,95 Gew.% |
| cis-Buten-2 | 3,15 Gew.% |
| Butadien-1,3 | 42,31 Gew.% |
| Butadien-1,2 | 0,10 Gew.% |
| Butin-1 | 0,11 Gew.% |
| Butenin | 0,36 Gew.% |

0022510

Eine Mischung von 457 g je Stunde dieses Kohlenwasserstoffgemisches mit 320 ml je Stunde Isobutanol wurde wie in Beispiel 1 beschrieben umgesetzt. Der Isobutengehalt im nach der Destillation erhaltenen Buten−/Butan−Raffinat lag bei 1,0 Gewichtsprozent.

Das Sumpfprodukt der ersten Destillation wurde verdampft und dann in einen rohrförmigen Spaltreaktor eingeleitet, in dem der Isobutyl-tert.-butyläther bei 190°C in Isobuten und Isobutanol aufgespalten wurde. Das am Kopf der nachgeschalteten Destillationsstufe abgezogene Isobuten (107 g je Stunde) hatte die folgende Zusammensetzung:

| | | |
|---|---|---|
| Butan | 0,012 | Gew.% |
| Isobutan | 0,041 | Gew.% |
| Buten−1 | 0,042 | Gew.% |
| Isobuten | 99,332 | Gew.% |
| trans−Buten−2 | 0,09 | Gew.% |
| cis−Buten−2 | 0,11 | Gew.% |
| Butadien−1,3 | 0,36 | Gew.% |
| Butadien−1,2 | 0,007 | Gew.% |
| Butin−1 | 0,0032 | Gew.% |
| Butenin | 0,0028 | Gew.% |

Trotz eines Butadien−1,3-Gehaltes im Ausgangs-$C_4$-Kohlenwasserstoffgemisch von 42,31 Gewichtsprozent betrug der Butadien−1,3-Gehalt im Isobuten−Produkt lediglich 0,36 Gewichtsprozent. Ebenso wurden auch Butadien−1,2 sowie Butin−1 und Butenin sehr stark abgereichert.

Die Ausbeute an Isobuten bezogen auf das im eingesetzten $C_4$-Kohlenwasserstoffgemisch enthaltene Isobuten betrug 96,5 %. Das am Sumpf der zweiten Destillationskolonne praktisch vollständig zurückgewonnene Isobutanol wurde wieder in die Verätherungsreaktion zurückgeführt.

0022510

Beispiel 3

Die Verätherung wurde unter Verwendung eines $C_4$-Kohlenwasserstoffgemisches durchgeführt, welches den Rest (Raffinat) einer aus einer Äthylenanlage erhaltenen $C_4$-Fraktion darstellte, aus der das Butadien extrahiert worden war. Die Zusammensetzung des $C_4$-Kohlenwasserstoffgemisches nach der Butadien-Extraktion war wie folgt:

| | |
|---|---|
| Isobutan | 1,9 Vol.% |
| n-Butan | 8,1 Vol.% |
| Isobuten | 46,0 Vol.% |
| Buten-1 | 26,7 Vol.% |
| trans-Buten-2 | 10,1 Vol.% |
| cis-Buten-2 | 7,0 Vol.% |
| Butadien | 0,2 Vol.% |

Eine Mischung von 250 g je Stunde dieses $C_4$-Kohlenwasserstoffgemisches mit 266 ml je Stunde n-Propanol wurde in ein rohrförmiges Reaktionsgefäß aus rostfreiem Stahl eingeleitet, in dem sich 138 ml eines sulfonierten Polystyroldivinylbenzol-harzes in der Wasserstofform (Lewatit SPC 118, Kornfraktion 0,8 bis 1 mm) befanden. In dem Reaktionsgefäß wurde eine Temperatur von 40°C und ein Druck von 12 bar aufrechterhalten. Das erhaltene Reaktionsgemisch wurde einer Destillationskolonne zugeführt, wobei am Kopf der Kolonne ein Buten-/Butan-Raffinat mit einem Isobutengehalt von 2 Gewichtsprozent erhalten wurde. Das Raffinat war praktisch Propanol-frei und konnte daher ohne weitere Reinigungsoperationen, z.B. ohne Einschaltung einer Wasserwäsche, unmittelbar als Ausgangsstoff für weitere Reaktionen verwendet werden. Am Sumpf der Destillationskolonne wurden 428 ml je Stunde Propyl-tert.-butyläther, der noch 27 Gewichtsprozent, bezogen auf das Gemisch, im Überschuß zugesetztes Propanol enthielt, abgezo-

gen und in einen Verdampfer eingeleitet. Der verdampfte, auf 170°C erhitzte Propyl-tert.-butyläther wurde zur Ätherspaltung in einen rohrförmigen Spaltreaktor, welcher als Spaltkatalysator 20 % $H_3PO_4$ auf Kieselgel (getempert) enthielt, eingeleitet und bei 180 bis 200°C in Isobuten und Propanol aufgespalten. Das Reaktionsprodukt der Ätherspaltung wurde in eine zweite Destillationskolonne eingeleitet, in der am Kopf 95,7 g je Stunde hochreines Isobuten der folgenden Zusammensetzung erhalten wurde:

| | | |
|---|---|---|
| Isobuten | 99,9 | Gew.% |
| Isobuten | 300 | Gew. ppm |
| Butan | 100 | Gew. ppm |
| Buten-1 | 100 | Gew. ppm |
| trans-Buten-2 | 100 | Gew. ppm |
| cis-Buten-2 | 100 | Gew. ppm |
| Butadien-1,3 | 100 | Gew. ppm |

Am Sumpf der zweiten Destillationskolonne wurden 287 ml je Stunde Propanol erhalten, das noch 7,2 % Propyl-tert.-butyläther enthielt. Dieses Gemisch konnte in die Verätherungsreaktion rückgeführt werden, wobei sich die erhaltene Isobuten-Menge auf 112 g je Stunde steigern ließ.

Die Ausbeute an Isobuten, bezogen auf das im eingesetzten $C_4$-Kohlenwasserstoffgemisch enthaltene Isobuten, betrug 97,5 %, wenn das gesamte Sumpfprodukt der zweiten Destillationskonne rückgeführt wurde.

Patentansprüche

1. Verfahren zur Gewinnung von Isobuten aus Isobuten enthaltenden C$_4$-Kohlenwasserstoffgemischen durch Umsetzung des Gemisches mit einem primären Alkohol in Gegenwart eines sauren Kondensationsmittels und Zerlegung des gebildeten tertiären Äthers in Gegenwart eines sauren Katalysators bei erhöhten Temperaturen, dadurch gezeichnet, daß man als primären Alkohol einen primären C$_3$- oder C$_4$-Alkohol und als saures Kondensationsmittel für die Ätherbildung Ionenaustauscher in der Wasserstofform verwendet, wobei man den primären C$_3$- oder C$_4$-Alkohol und das C$_4$-Kohlenwasserstoffgemisch, gegebenenfalls nach vorheriger Vermischung, zunächst der Reaktionszone für die Verätherung, in der sich der Ionenaustauscher befindet, zuführt, das aus der Reaktionszone für die Verätherung erhaltene Reaktionsgemisch anschließend in einer ersten Destillationszone destilliert, als Kopfprodukt ohne Einschaltung einer Wasserwäsche ein die nicht umgesetzten Kohlenwasserstoffe enthaltendes C$_4$-Kohlenwasserstoffgemisch mit einem Gehalt an primären C$_3$- oder C$_4$-Alkohol von höchstens 1000 Gewichts-ppm und als Bodenprodukt den gebildeten, gegebenenfalls noch im Überschuß zugesetzten primären C$_3$- oder C$_4$-Alkohol enthaltenden C$_3$- oder C$_4$-Alkyl-tert.-butyläther abzieht, das Bodenprodukt danach einer zweiten, einen sauren Katalysator enthaltenden Reaktionszone zuführt, in der der C$_3$- oder C$_4$-Alkyl-tert.-butyläther bei erhöhter Temperatur in Isobuten und primären C$_3$- oder C$_4$-Alkohol zerlegt wird, das Gemisch aus Isobuten und primären C$_3$- oder C$_4$-Alkohol einer zweiten Destillationszone zuführt, in der als Kopfprodukt

0022510

Isobuten ohne Einschaltung einer Wasserwäsche mit einem Gehalt an primärem $C_3$- oder $C_4$-Alkohol von höchstens 500 Gewichts-ppm und als Bodenprodukt der primäre $C_3$- oder $C_4$-Alkohol abgezogen werden, und den erhaltenen primären $C_3$- oder $C_4$-Alkohol in die Reaktionszone für die Verätherung zurückführt.

2. Verfahren nach Anspruch 1, dadurch gekennzeichnet, daß die Austrittstemperatur des Reaktionsgemisches aus der Reaktionszone für die Verätherung im Bereich von 25 bis 65°C liegt.

3. Verfahren nach Anspruch 1 und 2, dadurch gekennzeichnet, daß in der Verätherungsreaktion das im $C_4$-Kohlenwasserstoffgemisch enthaltene Isobuten zu mindestens 90 % zum $C_3$- oder $C_4$-Alkyl-tert.-butyläther umgesetzt wird.

4. Verfahren nach Anspruch 1 bis 3, dadurch gekennzeichnet, daß die Verätherungsreaktion kontinuierlich durchgeführt wird, wobei das Verhältnis aus dem Volumen der Reaktionszone für die Verätherung und dem Durchsatz an dem Isobuten enthaltenden $C_4$-Kohlenwasserstoffgemisch und dem primären $C_3$- oder $C_4$-Alkohol 0,01 bis 5 Stunden beträgt.

5. Verfahren nach Anspruch 1 bis 4, dadurch gekennzeichnet, daß man als Kopfprodukt der ersten Destillationszone ein die nicht umgesetzten Kohlenwasserstoffe enthaltendes $C_4$-Kohlenwasserstoffgemisch mit einem Gehalt von höchstens 200 Gewichts-ppm $C_3$- oder $C_4$-Alkyl-tert.-butyläther und/oder Di-$C_3$-alkyl- oder Di-$C_4$-alkyläther abzieht.

6. Verfahren nach Anspruch 1 bis 5, dadurch gekennzeichnet, daß man aus der ersten Destillationszone, ein den gebildeten $C_3$- oder $C_4$-Alkyl-tert.-butyläther enthaltendes Bodenprodukt mit einem Gehalt an $C_4$-Kohlenwasserstoffen von höchstens 1000 Gewichts-ppm abzieht.

7. Verfahren nach Anspruch 1 bis 6, dadurch gekennzeichnet, daß das aus der ersten Destillationszone erhaltene, den gebildeten $C_3$- oder $C_4$-Alkyl-tert.-butyläther enthaltende Bodenprodukt ohne Abtrennung von im Bodenprodukt enthaltenem primärem $C_3$- oder $C_4$-Alkohol als Ausgangsprodukt für die Zerlegungsstufe eingesetzt wird.

8. Verfahren nach Anspruch 1 bis 7, dadurch gekennzeichnet, daß die Zerlegung des $C_3$- oder $C_4$-Alkyl-tert.-butyläthers bei Drücken von 2 bis 15 bar durchgeführt wird.

9. Verfahren nach Anspruch 1 bis 8, dadurch gekennzeichnet, daß in der zweiten Destillationszone als Kopfprodukt Isobuten ohne Einschaltung einer Wasserwäsche mit einer Reinheit von mindestens 99,3 Gew.% und einem Gehalt an Di-$C_3$-alkyl- oder Di-$C_4$-alkyläther und/oder $C_3$- oder $C_4$-Alkyl-tert.-butyläther von höchstens 100 Gewichts-ppm abgezogen wird.

10. Verfahren nach Anspruch 1 bis 9, dadurch gekennzeichnet, daß der Gehalt des in die Reaktionszone für die Verätherung zurückgeführten primären $C_3$- oder $C_4$-Alkohols an gegebenenfalls in sehr kleinen Mengen als Nebenprodukt gebildetem, sich im zurückgeführten primären $C_3$- oder $C_4$-Alkohol anreicherndem Di-$C_3$-

-Alkyl- oder Di-C$_4$-Alkyläther auf 2 bis 20 Gew.% begrenzt wird.

11. Verfahren nach Anspruch 1 bis 10, dadurch gekennzeichnet, daß bei Verwendung von Isobutanol als primärem C$_4$-Alkohol als Seitenabzug aus der zweiten Destillationszone oder aus dem Bodenprodukt der zweiten Destillationszone ein kleiner Isobutanol-Teilstrom abgezweigt wird, der bei erhöhter Temperatur in Gegenwart eines Dehydratisierungskatalysators dehydratisiert wird.

12. Verfahren nach Anspruch 11, dadurch gekennzeichnet, daß ein 3 bis 40 Gew.% Diisobutyläther enthaltender Isobutanol-Teilstrom abgezweigt wird.

0022510

0022510

Nummer der Anmeldung

## Europäisches Patentamt

# EUROPÄISCHER RECHERCHENBERICHT

EP 80 10 3718.5

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| D | DE - B2 - 1 934 422 (SHELL INTERNATIO-NALE RESEARCH MAATSCHAPPIJ)<br>* Anspruch 1; Beispiele 9 und 10 *<br>-- | 1 |
| | DE - A1 - 2 752 111 (NIPPON OIL)<br>* Anspruch 1 *<br>-- | 1 |
| D | DE - A1 - 2 629 769 (CHEMISCHE WERKE HÜLS)<br>* Anspruch 1 *<br>-- | 1-5 |
| A | DE - A1 - 2 649 623 (MAGYAR ASVANYOLAJ ES FÖLDGAZ KISERLETI INTEZET)<br>* Anspruch 1 *<br>-- | 1 |
| P | DE - A1 - 2 802 199 (BASF)<br>* Ansprüche 1 bis 4 *<br>-- | 1,11,5 |
| P | EP - A2 - 0 003 305 (BASF)<br>* Ansprüche 1 bis 4 * | 1,11 |

----

### KLASSIFIKATION DER ANMELDUNG (Int.Cl.3)

C 07 C 11/09
C 07 C 7/148
C 07 C 1/24
C 07 C 41/06

### RECHERCHIERTE SACHGEBIETE (Int. Cl.3)

C 07 C 1/24
C 07 C 7/148
C 07 C 11/08
C 07 C 11/09
C 07 C 41/06

### KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 21-10-1980 | KNAACK |

EPA form 1503.1 06.78